# EUROPEAN PATENT APPLICATION

(11) **EP 2 583 945 A1**
(43) Date of publication of application: **24.04.2013**
(21) Application number: 12186719.6
(22) Date of filing: 28.09.2012
(51) Int. Cl.: C01G 23/047, H01L 49/02

(54) **MIMCAP structure comprising doped rutile titanium oxide and method of forming**

(30) Priority: 19.10.2011 US 201161548929 P
(71) Applicant: IMEC, 3001 Leuven (BE)
(72) Inventor: Popovici, Mihaela Ioana, 3001 Leuven (BE)
(74) Representative: Sarlet, Steven Renaat Irène

(57) **Abstract**

An electronic device includes a first electrode, and a layer of a dielectric material including titanium oxide and a first dopant ion. The layer of the dielectric material is formed on the first electrode. The first dopant ion has a size mismatch of 10% or lower compared to the Ti⁴⁺ ion and the dielectric material has a rutile tetragonal crystalline structure at temperatures below 650°C. The electronic device includes a second electrode, formed upon the dielectric material layer.

## Description

### Technical field

The present disclosure relates to a method for forming a MIMCAP structure and the MIMCAP structure thereof

### Background art

For future scaling of a Dynamic Random Access Memory Metal-Insulator-Metal Capacitors (DRAM MIMCAP), Titanium Dioxide (TiO₂) appears as an interesting dielectric layer due to relatively high k value when crystallized as rutile phase (at least 80) as compared to anatase phase (about 40). Ruthenium Dioxide (RuO₂) is a conductive oxide used both as bottom electrode and template for the growth of rutile TiO₂ at low temperature. It would be of great interest to avoid the use of RuO₂ which presents several inconveniences such as thermal stability and high price and replace it with alternative electrodes. However, in the absence of a template, rutile TiO₂ can be obtained only after anneals at high temperatures (about 900°C). Therefore a solution is needed to lower the temperature of rutile TiO₂ formation.

For future scaling of a DRAM MIMCAP not only a high dielectric constant dielectric is required, but also to find electrodes that do not further oxidize to form detrimental interfacial layers.

### Summary of the disclosure

It is an aim of the present disclosure to provide a MIMCAP structure with a high dielectric constant dielectric which does not further oxidize to form detrimental interfacial layers.

It is another aim of the present disclosure to provide a low temperature method for forming such MIMCAP structures.

These aims are achieved according to this disclosure by means of the subject-matter of the independent claims.

Disclosed herein are improved methods, systems, and devices for forming a MIMCAP structure and the MIMCAP structure thereof.

In one embodiment, an electronic device comprises a stack that includes a first electrode, and formed thereupon a layer of a dielectric material comprising titanium oxide and a first dopant ion. The first dopant ion has a size mismatch of 10% or lower compared to the Ti⁴⁺ ion and the dielectric material has a rutile tetragonal crystalline structure at temperatures below 650°C. The electronic device comprises a second electrode, formed upon the dielectric material layer.

In another embodiment, a method for manufacturing an electronic device comprises forming a first electrode, and thereupon forming a layer of a dielectric material comprising titanium oxide and a first dopant ion. The first dopant ion has a size mismatch of 10% or lower as compared to the Ti⁴⁺ ion wherein the dielectric material has a rutile tetragonal crystalline structure at temperatures below 650°C. The method further comprises forming a second electrode upon the dielectric material layer.

Anatase TiO₂ is kinetically favored even though rutile TiO₂ is the thermodynamically most stable phase. Therefore, prior art deposition of thin films at low temperatures or after anneals up to 600°C result in the anatase phase formation. However, for use as a dielectric layer, the rutile phase is preferred because of its significantly higher dielectric constant. But then a higher thermal budget is needed in the prior art techniques to fully convert anatase to rutile (about 900°C).

Both anatase and rutile have a tetragonal structure, the difference residing in the cell parameters respectively the volume of the cell which is smaller for rutile TiO₂.

The present application discloses forming rutile TiO₂ by doping TiO₂ with a cation having a smaller radius. It is believed that the cation enters into the lattice of TiO₂ to replace the Ti⁴⁺ site and consequently, the cell contracts. One of the suitable cations whose radius (size) is only slightly smaller than that of Ti⁴⁺ is Mg²⁺. Alternatively, other dopants such as Co²⁺, Ni²⁺, Zn²⁺, V²⁺ are also suitable. Dopants having a radius smaller or slightly larger (a mismatch in radius of 10% or lower) than that of the titanium atom are also suitable.

Without wishing to be bound by theory it is believed that processes that create oxygen vacancies, such as the addition of acceptor dopants (ions with a lower valence that Ti⁴⁺) and use of reducing atmospheres accelerate the anatase-rutile transformation. Conversely, processes that increase the concentration of titanium interstitials, such as the addition of donor dopants, inhibit the transformation.

In a preferred embodiment of the electronic device, at least one of the first and the second electrode comprises a metal or a conductive oxide, wherein the wherein the conductive oxide is a material suitable to be used as transparent conductive oxide (TCO).

In a preferred embodiment of the electronic device, the concentration of the first dopant ion lies between 0.3 %at and 20 %at.

In a preferred embodiment of the method for manufacturing the electronic device, the conductive oxide is a material suitable to be used as transparent conductive oxide (TCO).

### Brief description of the drawings

The disclosure will be further elucidated by means of the following description and the appended figures.

All drawings are intended to illustrate some aspects and embodiments of the present disclosure. The drawings described are only schematic and are non-limiting.
Figure 1 shows the Grazing Incidence X-ray diffraction (GIXRD) pattern of a O₃ based TiO₂ film deposited by Atomic Layer Deposition (ALD) and annealed at 600°C 1 minute in O₂ containing ambient as a reference for rutile-TiO_{2;}
Figure 2A shows the GIXRD pattern of Ti reference films as deposited by Physical Vapor Deposition (PVD) and after post-deposition anneal at 600°C 1 minute in O₂ containing ambient; and
Figure 2B shows the GIXRD pattern of Mg doped TiO₂ (16.5% at. Mg) as deposited by Physical Vapor Deposition (PVD) and after post-deposition anneal at 600°C 1 minute in O₂ containing ambient.

### Detailed description of preferred embodiments

The present disclosure will be described with respect to particular embodiments and with reference to certain drawings but the disclosure is not limited thereto but only by the claims. The drawings described are only schematic and are non-limiting. In the drawings, the size of some of the elements may be exaggerated and not drawn on scale for illustrative purposes. The dimensions and the relative dimensions do not necessarily correspond to actual reductions to practice of the disclosure.

Furthermore, the terms first, second, third and the like in the description and in the claims, are used for distinguishing between similar elements and not necessarily for describing a sequential or chronological order. The terms are interchangeable under appropriate circumstances and the embodiments of the disclosure can operate in other sequences than described or illustrated herein.

Moreover, the terms top, bottom, over, under and the like in the description and the claims are used for descriptive purposes and not necessarily for describing relative positions. The terms so used are interchangeable under appropriate circumstances and the embodiments of the disclosure described herein can operate in other orientations than described or illustrated herein.

Furthermore, the various embodiments, although referred to as "preferred" are to be construed as exemplary manners in which the disclosure may be implemented rather than as limiting the scope of the disclosure.

The term "comprising", used in the claims, should not be interpreted as being restricted to the elements or steps listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a device comprising A and B" should not be limited to devices consisting only of components A and B, rather with respect to the present disclosure, the only enumerated components of the device are A and B, and further the claim should be interpreted as including equivalents of those components.

In one aspect, an electronic device comprising the following stack is disclosed:
- a first electrode, and thereupon
- a layer of a dielectric material comprising titanium oxide and a first dopant ion, wherein the first dopant ion has a size smaller or slightly larger than the Ti⁴⁺ ion and the dielectric material has a rutile tetragonal crystalline structure at temperatures below 650°C, and thereupon
- a second electrode.

The first dopant ion is chosen such as in the octahedral coordination characteristic to the rutile structure it has a radius mismatch of 10% or lower compared to the Ti⁴⁺ ion. In embodiments of the disclosure the first dopant ion can selected from the group consisting of Mg, Sc, Co, Ni, Zn, V, Cr, Cu, Zr, Nb, Mo, Ru, Rh and any combinations thereof.

The minimum concentration necessary to trigger the formation of rutile TiO₂ depends on the dopant ion selected. In some embodiments about 0.3%at of dopant ion can be enough to lead to the formation of rutile TiO₂. Higher concentrations of 1%at or more will also lead to the formation rutile TiO₂. The dopant ion concentration preferably does not exceed an upper limit that leads to phase segregation, i.e. in specific examples about 20 %at.

In some embodiments of the disclosure the dielectric material may comprise further strontium. However, in these specific examples the structure formed upon crystallization is of perovskite type. Both rutile TiO₂ and perovskite STO containing dopant elements are dielectrics characterized by high-k values and large band gap suitable for DRAM MIMCAP applications.

In embodiments of the disclosure at least one of the first and the second electrode comprises a metal or a conductive oxide.

The first and/or the second electrode may comprise a metal such as TiN, TaN, Ru, Ni, Mo, W or any combinations thereof.

In alternative embodiments of the disclosure the conductive oxide comprises a first metal oxide. Non limiting examples of first metal oxides having a suitable resistivity (lower than 10⁻³ ohm.cm) are RuO₂, IrO₂, MoO₂, VO₂, In₂O₃ or any combinations thereof.

In yet other embodiments of the disclosure the conductive oxide comprises a second metal oxide and a second dopant. The second metal oxide is selected from the group consisting of In₂O₃, SnO₂, CdO, MgO, ZnO and any combinations thereof. The second dopant is selected from the group consisting of Sn, Sb, Ti, Y, Mo, Zr, W, Al and any combinations thereof.

In particular embodiments the conductive oxide is any material suitable to be used as transparent conductive oxide (TCO).

In a second aspect, a method for manufacturing an electronic device is disclosed comprising:
- Forming a first electrode, and thereupon
- Forming a layer of a dielectric material comprising titanium oxide and a first dopant ion, wherein the first dopant ion has a radius mismatch of 10% or lower as compared to Ti⁴⁺ ion radius, wherein the dielectric material has a rutile tetragonal crystalline structure at temperatures below 650°C and thereupon
- Forming a second electrode

In different embodiments of the disclosure at least one of the layers of dielectric material and the first or the second electrode is formed by Atomic Layer Deposition or by Chemical Vapor Deposition (CVD).

In some embodiments of the second aspect the first dopant ion is selected from the group consisting of Mg, Sc, Co, Ni, Zn, V, Cr, Cu, Zr, Nb, Mo, Ru, Rh and any combinations thereof.

In other embodiments of the second aspect the dielectric material may comprise further strontium. However, in these specific examples the structure of the dielectric material is of perovskite type.

In different embodiments of both aspects any of the layer of dielectric material and the first or the second electrode may comprise multiple sub-layers having the same of a different composition. Alternatively, any of the layer of dielectric material and the first or the second electrode may be a single layer having a homogeneous or a graded composition.

The dielectric material has a rutile tetragonal crystalline structure in the as-deposited form or, alternatively, the rutile structure is formed upon applying an additional thermal treatment after depositing the layer of dielectric material. In particular embodiments the thermal treatment is a post deposition anneal performed in-situ (in the deposition chamber) or ex-situ in oxidizing atmosphere at a temperature below 650°C.

Embodiments of the second aspect disclose that at least one of the first and the second electrode comprises a metal or a conductive oxide.

In some embodiments the conductive oxide comprises a first metal oxide selected from the group consisting of RuO₂, IrO₂, MoO₂, VO₂, In₂O₃ or any combinations thereof.

In alternative embodiments the conductive oxide comprises a second metal oxide and a second dopant. Non limitative examples of the second metal oxide are selected from the group consisting of In₂O₃, SnO₂, CdO, MgO, ZnO and any combinations thereof. The second dopant is selected from the group consisting of Sn, Sb, Y, Ti, Mo, Zr, W, Al and any combinations thereof.

In particular embodiments the conductive oxide is any material suitable to be used as transparent conductive oxide (TCO).

### Examples

In one embodiment the formation of dielectric rutile TiO₂ doped with Mg on 300 mm wafer starting from a Physical Vapor Deposition (PVD) TiMg co-sputtered film is disclosed. Before performing the thermal treatments (anneals) in an oxidant atmosphere, the films were capped with 2 nm TiO₂ ALD deposited at 250°C using titanium methoxide and O₃ as reactants. However, the cap layer is optional. As a reference, a thick TiO₂ film (14 nm) was deposited by ALD and thereafter annealed for 1 min in O₂ at 600°C.

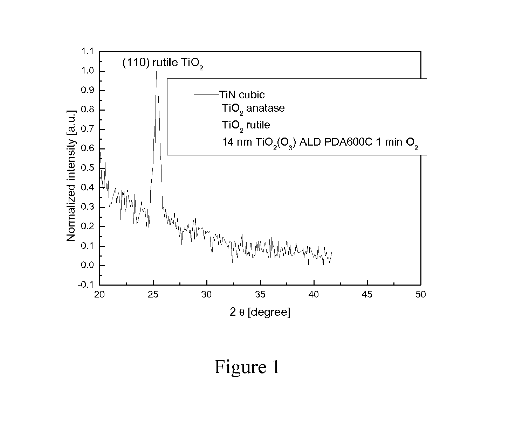

The GIXRD pattern of the reference sample in Figure 1 showed the formation of anatase phase.

In different examples Ti was deposited by co-sputtering together with Mg from Ti and Mg sources at 0%, 11%, 13% and 16.5 % Mg concentration.

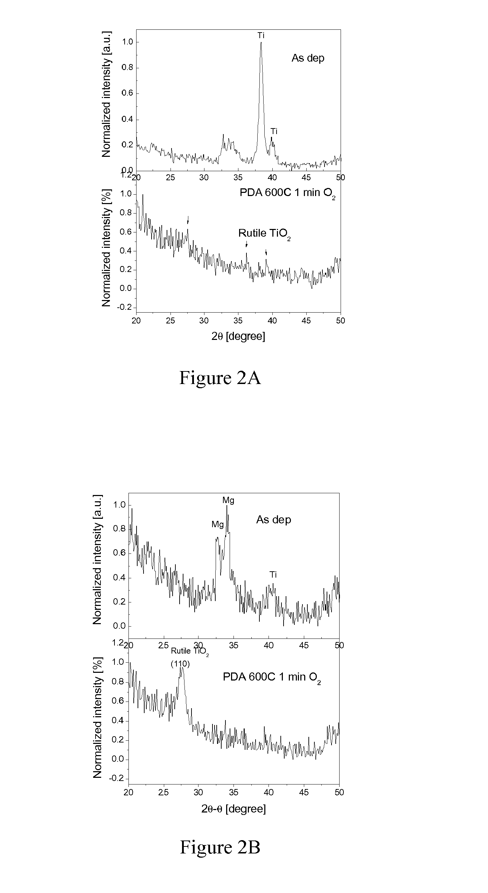

In case of the as deposited films, the reference Ti sample was well crystallized (Ti cubic and Ti hexagonal), while the Mg doped samples showed the presence of weakly crystallized hexagonal Ti together with crystalline Mg (Figure 2a).

After capping with TiO₂ and post-deposition anneal at 600C 1 min in O₂, the GIXRD showed the formation of weakly crystallized TiO₂ for undoped samples, while doped samples showed formation of a well crystallized rutile TiO₂. No Mg, or MgO was detected, likely the Mg has been at least partially incorporated in the rutile TiO₂ structure (Figure 2b). Preferably, the dopant ion (e.g. Mg) is completely or substantially completely incorporated in the rutile TiO₂ structure.

In alternative embodiments Mg doped rutile TiO₂ is formed by ALD or CVD. Advantageously, the tuning of the Mg concentration is easier to accomplish in an ALD or CVD process.

A further advantage of the ALD route is that it can be combined with the formation of a conductive oxide (e.g. a TCO) or a metal as electrode also by ALD. An example of such a conductive oxide is Ti doped MgO.

The particular combination of Ti doped MgO as electrode and Mg doped TiO₂ as dielectric having a rutile structure (and therefore a high k value and a large band gap) is advantageous and easily to implement in an ALD sequence. Alternative conductive oxides are Ti doped CdO or Ti doped In₂O₃, each of them having a resistivity below 10⁻³ Ohm.cm.

In a further aspect a MIMCAP device comprising a first electrode made of a conductive oxide and thereupon a doped TiO₂ having a rutile structure and thereupon a second electrode is disclosed. The first electrode and the second electrode can be made of the same conductive material or not. The conductive material of the first and/or the second electrode is either a metal or a conductive metal oxide. Preferably the conductive metal oxide comprises further dopants. The stack of the disclosure can be comprised alternatively in another device such e.g. a RRAM memory.

In another embodiment, Mg doped TiO₂ films were deposited by ALD using bis-methylcyclopentadienyl magnesium Mg(MeCp)₂ and Ti(OCH₃)₄ as precursors. The reaction cycle is {[H₂O/Ti(OCH₃)₄]ₓ[/H₂O/Mg(MeCp)₂]_{y}/[H₂O/Ti(OCH₃)₄]_{z}}n or {[O₃/Ti(OCH₃)₄]ₓ[/H₂O/Mg(MeCp)₂]_{y}/[O₃/Ti(OCH₃)₄]_{z}}-n.

The sequence of x, y or z sub-cycles can vary as follows: (x/y/z)n, or when x=z (y/x)n; (x/y)n. Three particular situations are considered for a total number of 222 cycles of O₃/Ti(OCH₃)₄ and 3 cycles of /H₂O/Mg(MeCp)₂ deposited at 250°C with a targeted thickness of about 12 nm Mg doped TiO₂.
- (1) Mg is uniformly distributed, i.e. 1 cycle of Mg is provided at the bottom of the film, in the middle of the film and at the top of the film;, {[O₃/Ti(OCH₃)₄]₇₄[/H₂O/Mg(MeCp)₂]₁}·3
- (2) 3 cycles of Mg are provided in the middle of the film: {[O₃/Ti(OCH₃)₄]₁₁₁[/H₂O/Mg(MeCp)₂]₁[O₃/Ti(OCH₃)₄]₁₁₁}·1
- (3) 3 cycles of Mg are provided at the top of the film: {[O₃/Ti(OCH₃)₄]₂₂₂[/H₂O/M_{g}(MeCp)₂]₁}·1.

All dielectric films formed as disclosed above are crystalline and have textured rutile structure (110).

The EOT-Jg data (EOT=equivalent oxide thickness and Jg=leakage current density) were recorded for the three MIM capacitors in which the Mg doped TiO₂ is the dielectric of the stack consisting of 10 nm TiN/5 nm Ru(as bottom electrode), 12 nm Mg doped TiO₂ (as dielectric) and 40 nm TiN (as top electrode). When the dependence EOT- Jg is given for +1V polarity, the Jg increases when MgO is present at the top of the stack as compared to reference (pure TiO₂).

In spite of insertion of 3 consecutive MgO cycles in the middle of the TiO₂ layer crystallization is not interrupted and a low Jg (of about 1x10⁻⁷ A/cm²) is obtained comparable with the pure TiO₂.

Similar EOT and Jg values are obtained when MgO is deposited in the middle of the stack as 1 cy MgO or 3 cycles MgO as compared to pure TiO₂. The 3 cycle MgO in fact showed slightly lower EOT pointing to a contraction of the oxide lattice and increase in the dielectric constant with the addition of MgO.

MgO does not contribute to the increase of the EOT in spite of a larger bandgap of about 7.3. eV -7.8 eV and low dielectric constant (9.8). This behaviour indicates the substitution of Ti⁴⁺ with Mg²⁺ in the rutile lattice than separate layer formation. An improvement at the negative polarity (lower Jg obtained at an applied voltage of -1V) is observed for larger Mg content.

## Claims

1. An electronic device comprising:
a first electrode,
a layer of a dielectric material, formed on the first electrode, the dielectric material layer comprising titanium oxide and a first dopant ion, wherein the first dopant ion has a size mismatch of at most 10% compared to the Ti⁴⁺ ion and the dielectric material has a rutile tetragonal crystalline structure at temperatures below 650°C; and
a second electrode formed on the dielectric material layer.

2. The device of claim 1, wherein the first dopant ion is selected from the group consisting of: Mg, Sc, Co, Ni, Zn, V, Cr, Cu, Zr, Nb, Mo, Ru, Rh and any combinations thereof.

3. The device of claim 1, wherein at least one of the first and the second electrode comprises a metal or a conductive oxide.

4. The device of claim 3, wherein the conductive oxide comprises a first metal oxide selected from the group consisting of: RuO₂, IrO₂, MoO₂, VO₂, In₂O₃ and any combinations thereof.

5. The device of claim 3, wherein the conductive oxide comprises a second metal oxide and a second dopant.

6. The device of claim 5, wherein the second metal oxide is selected from the group consisting of: In₂O₃, SnO₂, CdO, MgO, ZnO and any combinations thereof and wherein the second dopant is selected from the group consisting of: Sn, Sb, Y,Ti, Mo, Zr, W, Al and any combinations thereof.

7. A method for manufacturing an electronic device comprising:
forming a first electrode,
forming, on the first electrode, a layer of a dielectric material comprising titanium oxide and a first dopant ion, wherein the first dopant ion has a size mismatch of 10% or lower as compared to the Ti⁴⁺ ion wherein the dielectric material has a rutile tetragonal crystalline structure at temperatures below 650°C; and
forming, on the dielectric material layer, a second electrode.

8. The method of claim 7, wherein at least one of the layer of dielectric material, the first electrode, and the second electrode is formed by Atomic Layer Deposition.

9. The method of claim 7 or 8, wherein the first dopant is selected from the group consisting of: Mg, Sc, Co, Ni, Zn, V, Cr, Cu, Zr, Nb, Mo, Ru, Rh and any combinations thereof.

10. The method of any one of the claims 7-9, wherein the dielectric material has a rutile tetragonal crystalline structure in the as-deposited form.

11. The method of any one of the claims 7- 9, wherein the dielectric material has a rutile tetragonal crystalline structure upon applying a thermal treatment at a temperature below 650°C, after depositing the layer of dielectric material.

12. The method of any one of the claims 7-11, wherein at least one of the first and the second electrode comprises a metal or a conductive oxide.

13. The method of claim 12, wherein the conductive oxide comprises a first metal oxide selected from the group consisting of: RuO₂, IrO₂, MoO₂, VO₂, In₂O₃ and any combinations thereof.

14. The method of claim 12, wherein the conductive oxide comprises a second metal oxide and a second dopant.

15. The method of claim 14, wherein the second metal oxide is selected from the group consisting of: In₂O₃, SnO₂, CdO, MgO, ZnO and any combinations thereof and wherein the second dopant is selected from the group consisting of: Sn, Sb, Y Ti, Mo, Zr, W, Al and any combinations thereof.
